(19) Europäisches Patentamt
European Patent Office
Office européen des brevets

(11) Veröffentlichungsnummer: **0 065 295**
**A1**

# (12) EUROPÄISCHE PATENTANMELDUNG

(21) Anmeldenummer: **82104244.7**

(22) Anmeldetag: **14.05.82**

(51) Int. Cl.³: **C 07 D 409/12**, A 61 K 31/40

(30) Priorität: **19.05.81 DE 3119796**

(43) Veröffentlichungstag der Anmeldung: **24.11.82 Patentblatt 82/47**

(84) Benannte Vertragsstaaten: **AT BE CH DE FR GB IT LI LU NL SE**

(71) Anmelder: **HOECHST AKTIENGESELLSCHAFT, Postfach 80 03 20, D-6230 Frankfurt/Main 80 (DE)**

(72) Erfinder: **Lessenich, Hans, Dr., Gimbacher Tann 30, D-6233 Kelkheim (Taunus) (DE)**
Erfinder: **Kunstmann, Rudolf, Dr., Auf der Ahl 37, D-6200 Wiesbaden (DE)**
Erfinder: **Lindner, Ernst, Prof.Dr., Amselweg 8, D-6230 Frankfurt am Main 80 (DE)**

## (54) Substituierte Tryptaminderivate von Thienyloxypropanolaminen, Verfahren zu ihrer Herstellung, pharmazeutische Präparate auf Basis dieser Verbindungen sowie ihre Verwendung.

(57) Racemische und optisch aktive, substituierte Tryptaminderivate von Thienyloxypropanolaminen der allgemeinen Formel I,

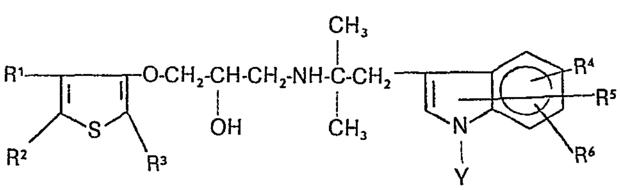

I

sowie deren Salze, Verfahren zur Herstellung dieser Verbindungen, ihre Verwendung bei der Behandlung von Herz-Kreislauferkrankungen und pharmazeutische Präparate auf Basis dieser Verbindungen.

EP 0 065 295 A1

Substituierte Tryptaminderivate von Thienyloxypropanolaminen, Verfahren zu ihrer Herstellung, pharmazeutische Präparate auf Basis dieser Verbindungen sowie ihre Verwendung

Die Erfindung betrifft substituierte Tryptaminderivate von Thienyloxypropanolaminen der allgemeinen Formel I,

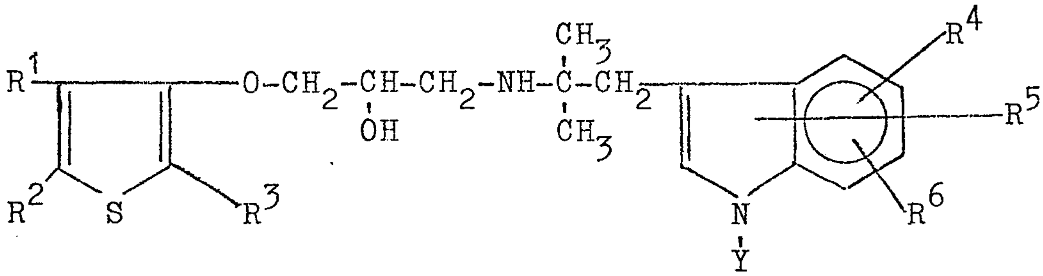

I

sowie deren physiologisch verträgliche Salze, die aufgrund ihrer pharmakologischen Eigenschaften als Arzneimittel verwendet werden können.

In der Formel bedeuten:

$R^1$, $R^2$ und $R^3$

a) Wasserstoff, Halogen, Alkyl mit 1 bis 6 C-Atomen, Alkoxy mit 1 bis 4 C-Atomen, Alkoxymethyl mit 1 bis 4 C-Atomen im Alkoxylteil, Hydroxymethyl,

b) Phenyl, gegebenenfalls mono-, di- oder trisubstituiert mit Halogen, $C_1$-$C_6$-Alkyl oder $C_1$-$C_4$-Alkoxy, oder Phenylalkyl mit 1 bis 4 C-Atomen im Alkylteil,

c) Cycloalkyl mit 3 bis 8-C-Atomen,

d) Cyano, Nitro, Amino,

e) Carboxyl, Alkoxycarbonyl mit 1 bis 4 C-Atomen im Alkoxylteil,

f) Carbonamido der Formel 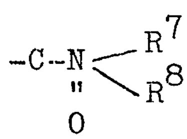

worin $R^7$ und $R^8$ gleich oder verschieden sind und Wasserstoff, Alkyl mit 1 bis 6 C-Atomen, oder Phenyl, gegebenenfalls substituiert mit $C_1$-$C_4$-Alkyl, $C_1$-$C_4$-Alkoxy oder Halogen, bedeuten oder

worin $R^7$ und $R^8$ Alkylreste sind, die gemeinsam mit dem Stickstoffatom einen 5- bis 7-gliedrigen Ring bilden, wobei eine $-CH_2$-Gruppe durch Sauerstoff, Schwefel oder die $NR^9$-Gruppe ersetzt sein kann, worin $R^9$ Wasserstoff, $C_1$-$C_4$-Alkyl, Phenyl oder Phenylalkyl mit 1 bis 2 C-Atomen im Alkylteil bedeutet,

g) $R^1$ und $R^2$ zusammen mit dem Thiophenring ein kondensiertes heteroaromatisches Ringsystem mit insgesamt 8 bis 9 Ringgliedern und 1 bis 2 Schwefelatomen, wobei $R^3$ die unter a) bis f) genannten Bedeutungen besitzt,

$R^4$, $R^5$ und $R^6$

a) Wasserstoff, Halogen, Alkyl mit 1 bis 6 C-Atomen, Alkoxy mit 1 bis 4 C-Atomen, Hydroxy,

b) Phenyl, gegebenenfalls mono- oder disubstituiert mit $C_1$-$C_4$-Alkyl oder $C_1$-$C_4$-Alkoxy oder Halogen,

c) Nitro, Amino,

d) Carboxyl, Alkoxycarbonyl mit 1 bis 4 C-Atomen im Alkoxylteil oder Carbonamido der Formel $-C(=O)-N<^{R^{10}}_{R^{11}}$

worin $R^{10}$ und $R^{11}$ gleich oder verschieden sind und Wasserstoff oder Alkyl mit 1 bis 6 C-Atomen bedeuten, wobei die Alkylreste gemeinsam mit dem Stickstoffatom einen 5- bis 7-gliedrigen Ring bilden können, worin eine $-CH_2$-Gruppe durch Sauerstoff, Stickstoff oder die $NR^{12}$-Gruppe, worin $R^{12}$ Wasserstoff, $C_1$-$C_4$-Alkyl, Phenyl oder Phenylalkyl mit 1 bis 2 C-Atomen im Alkylteil bedeutet, ersetzt sein kann,

Y-Wasserstoff, $C_1$-$C_4$-Alkyl oder Phenyl.

Die Substituenten $R^1$, $R^2$ und $R^3$ bzw. $R^4$, $R^5$ und $R^6$ können gleich oder verschieden sein. Die Alkylreste sind geradkettig oder verzweigt. Der Substituent $R^5$ kann sich sowohl im isocyclischen als auch im heterocyclischen Ring des Indolsystems befinden.

Insbesondere beinhaltet die Erfindung Verbindungen der Formel I, worin $R^1$, $R^2$ und $R^3$ gleich oder verschieden sind und

a) Wasserstoff, Halogen, Alkyl mit 1 bis 4 C-Atomen, Alkoxy mit 1 bis 4 C-Atomen, Alkoxymethyl mit 1 bis 4 C-Atomen

im Alkoxylteil, Hydroxymethyl,

b) Phenyl, gegebenenfalls mono- oder disubstituiert mit Halogen, $C_1$-$C_4$-Alkyl oder $C_1$-$C_4$-Alkoxy, oder Benzyl,

c) Cycloalkyl mit 3 bis 8 C-Atomen,

d) Cyano, Nitro, Amino,

e) Carboxyl, Alkoxycarbonyl mit 1 bis 4 C-Atomen im Alkoxylteil,

f) Carbonamido der Formel $-\underset{O}{\underset{\|}{C}}-N\begin{matrix}R^7\\R^8\end{matrix}$

worin $R^7$ und $R^8$ gleich oder verschieden sind und Wasserstoff, Alkyl mit 1 bis 6 C-Atomen, oder Phenyl, gegebenenfalls substituiert mit $C_1$-$C_4$-Alkyl, $C_1$-$C_4$-Alkoxy oder Halogen, bedeuten oder worin $R^7$ und $R^8$ Alkylreste sind, die gemeinsam mit dem Stickstoffatom einen 5- bis 7-gliedrigen Ring bilden, wobei eine $-CH_2$-Gruppe durch Sauerstoff, Schwefel oder die $NR^9$-Gruppe ersetzt sein kann, worin $R^9$ Wasserstoff, $C_1$-$C_4$-Alkyl, Phenyl oder Phenylalkyl mit 1 bis 2 C-Atomen im Alkylteil bedeutet,

g) $R^1$ und $R^2$ zusammen mit dem Thiophenring ein kondensiertes heteroaromatisches Ringsystem mit insgesamt 8 bis 9 Ringgliedern und 1 bis 2 Schwefelatomen, wobei $R^3$ die unter a) bis f) genannten Bedeutungen besitzt,

$R^4$, $R^5$ und $R^6$ gleich oder verschieden sind und

a) Wasserstoff, Halogen, Alkyl mit 1 bis 4 C-Atomen, Alkoxy mit 1 bis 4 C-Atomen, Hydroxy,

b) Phenyl, gegebenenfalls mono- oder disubstituiert mit $C_1$-$C_4$-Alkyl, $C_1$-$C_4$-Alkoxy oder Halogen,

c) Nitro, Amino,

d) Carboxyl, Alkoxycarbonyl mit 1 bis 2 C-Atomen im Alkoxylteil oder Carbonamido der Formel $-\underset{O}{\underset{\|}{C}}-N\begin{matrix}H\\H\end{matrix}$

Y Wasserstoff, Methyl oder Phenyl bedeuten und deren Salze mit physiologisch verträglichen Säuren.

Von besonderem Interesse sind Verbindungen der Formel I, worin $R^1$, $R^2$ und $R^3$ gleich oder verschieden sind und

a) Wasserstoff, Methyl, Ethyl, Alkoxymethyl mit 1 bis 4

C-Atomen im Alkoxylteil,

b) Phenyl, gegebenenfalls mono- oder disubstituiert mit Methyl, Chlor, Fluor oder Methoxy, oder Benzyl,

c) Cycloalkyl mit 5 bis 7 C-Atomen,

d) Cyano,

e) Carboxyl, Alkoxycarbonyl mit 1 bis 4 C-Atomen im Alkoxylteil,

f) Carbonamido der Formel $-\underset{\substack{\| \\ O}}{C}-N\begin{smallmatrix} R^7 \\ R^8 \end{smallmatrix}$

worin $R^7$ und $R^8$ gleich oder verschieden sind und Wasserstoff, Alkyl mit 1 bis 6 C-Atomen, oder Phenyl, gegebenenfalls substituiert mit $C_1$-$C_4$-Alkyl, $C_1$-$C_4$-Alkoxy oder Halogen, bedeuten oder worin $R^7$ und $R^8$ Alkylreste sind, die gemeinsam mit dem Stickstoffatom einen 5- bis 7-gliedrigen Ring bilden, wobei eine $-CH_2$-Gruppe durch Sauerstoff, Schwefel oder die $NR^9$-Gruppe ersetzt sein kann, worin $R^9$ Wasserstoff, $C_1$-$C_4$-Alkyl, Phenyl oder Phenylalkyl mit 1 bis 2 C-Atomen im Alkylteil bedeutet,

g) $R^1$ und $R^2$ zusammen mit dem Thiophenring ein kondensiertes heteroaromatisches Ringsystem mit insgesamt 8 bis 9 Ringgliedern und 1 bis 2 Schwefelatomen, wobei $R^3$ die unter a) bis f) genannten Bedeutungen besitzt,

$R^4$, $R^5$ und $R^6$ gleich oder verschieden sind und

a) Wasserstoff, Brom oder Chlor, Alkyl mit 1 bis 2 C-Atomen, Alkoxy mit 1 bis 2 C-Atomen, Hydroxy,

b) Phenyl, gegebenenfalls mono- oder disubstituiert mit $C_1$-$C_4$-Alkyl, $C_1$-$C_4$-Alkoxy oder Halogen,

c) Nitro, Amino,

e) Carboxy, Methoxycarbonyl, Äthoxycarbonyl oder Carbonamido der Formel $-\underset{\substack{\| \\ O}}{C}-N\begin{smallmatrix} H \\ H \end{smallmatrix}$

Y Wasserstoff oder Methyl bedeuten und deren Salze mit physiologisch verträglichen Säuren.

Ganz besonders bevorzugt sind Verbindungen der Formel I und deren Salze, worin die verschiedenen Substituenten die

folgenden Bedeutungen haben:

$R^1$ und $R^2$: Wasserstoff, Methyl, Äthyl, Phenyl, Benzyl, wobei $R^1$ und $R^2$ gleich oder verschieden sind,

$R^3$: Cyano, Alkoxycarbonyl mit 1 bis 4 C-Atomen im Alkoxylteil, Carbonamido der Formel $-\underset{\underset{O}{\|}}{C}-N<^{R^7}_{R^8}$ worin $R^7$ und $R^8$ gleich oder verschieden sind und Wasserstoff, Alkyl mit 1 bis 4 C-Atomen, Phenyl, gegebenenfalls substituiert mit $C_1$-$C_4$-Alkyl oder $C_1$-$C_4$-Alkoxy, oder worin $R^7$ und $R^8$ Alkylreste sind, die gemeinsam mit dem N-Atom einen 5- bis 7-gliedrigen Ring bilden, wobei eine $-CH_2$-Gruppe durch Sauerstoff, Schwefel oder die NH-Gruppe ersetzt sein kann,

$R^4$, $R^5$ und $R^6$ Wasserstoff, Y Wasserstoff oder Methyl.

Verbindungen in dieser Strukturklasse zeigen im allgemeinen betasympatholytische Wirkung. Überraschenderweise wurde bei den erfindungsgemäßen Verbindungen gefunden, daß sie darüberhinaus im akuten Versuch aufgrund ihrer vasodilatierenden Eigenschaften ausgeprägt blutdrucksenkend wirken und damit wertvolle Arzneimittel darstellen.

Die Erfindung betrifft daher auch pharmazeutische Präparate auf Basis von Verbindungen der Formel I und ihrer Salze sowie ihre Verwendung zur Behandlung von Krankheiten, insbesondere Herz-Kreislauf-Erkrankungen.

Die Erfindung betrifft auch Verfahren zur Herstellung von Verbindungen der Formel I und deren Salzen. Sie sind dadurch gekennzeichnet, daß man

a) ein Epoxid der Formel II,

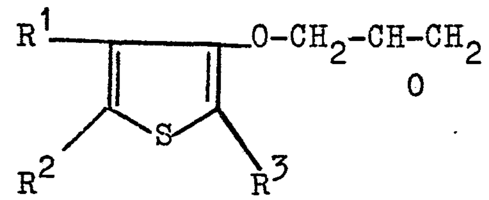

II

worin $R^1$, $R^2$ und $R^3$ die obige Bedeutung haben, mit einem Amin der Formel III,

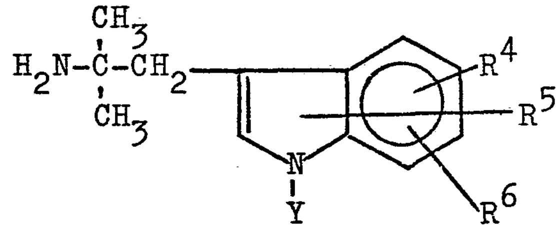

III

worin $R^4$, $R^5$, $R^6$ und Y die obige Bedeutung haben, umsetzt oder

b) eine Verbindung der Formel IV,

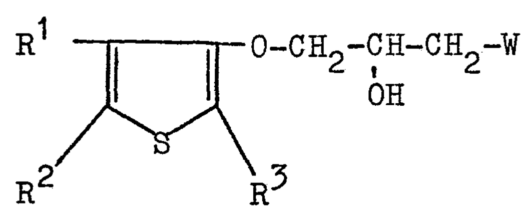

IV

worin $R^1$, $R^2$ und $R^3$ die obige Bedeutung haben und W eine durch nukleophile Substitution ersetzbare Gruppe darstellt, mit einem Amin der Formel III, worin $R^4$, $R^5$, $R^6$ und Y die obige Bedeutung haben, umsetzt, oder

c) ein Amin der Formel V,

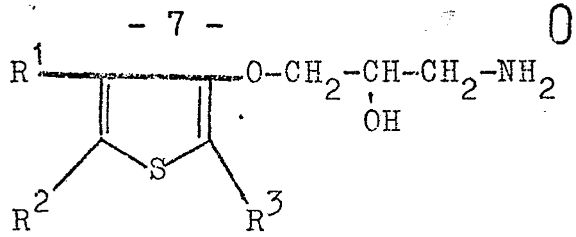

V

worin $R^1$, $R^2$ und $R^3$ die obige Bedeutung haben, mit einer Verbindung der Formel VI,

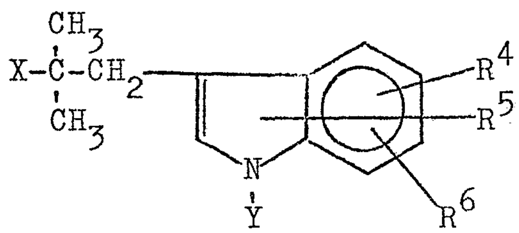

VI

worin $R^4$, $R^5$, $R^6$ und Y die obige Bedeutung haben und X eine durch nukleophile Substitution ersetzbare Gruppe darstellt, umsetzt, oder

d) ein Thiophenderivat der Formel VII,

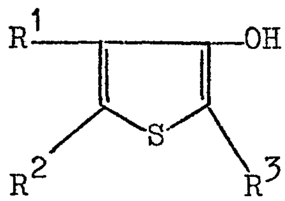

VII

worin $R^1$, $R^2$ und $R^3$ die obige Bedeutung haben, mit einer Verbindung der Formel VIII,

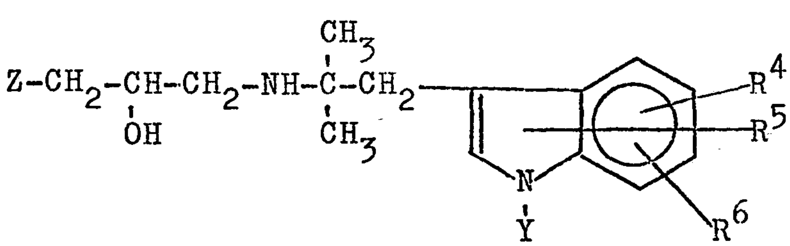

VIII

worin $R^4$, $R^5$, $R^6$ und Y die zu Formel I angegebene Bedeutung haben und Z eine durch nukleophile Substitution ersetzbare Gruppe darstellt, in Gegenwart eines säurebindenden Mittels umsetzt, oder

e) in einer Verbindung der Formel IX,

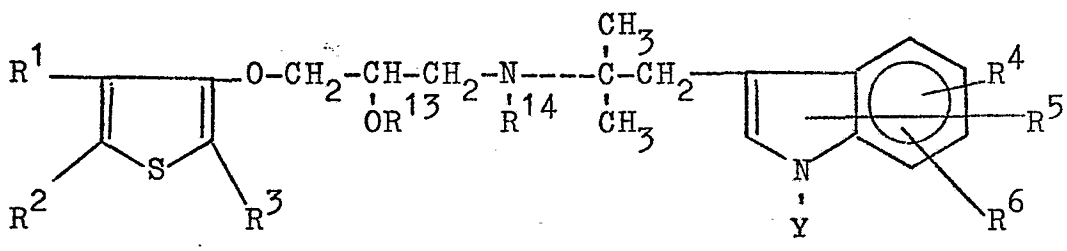

IX

worin $R^1$, $R^2$, $R^3$, $R^4$, $R^5$, $R^6$ und Y die obige Bedeutung haben,

$R^{13}$ ein Wasserstoffatom, einen Acylrest oder den Benzylrest und $R^{14}$ ein Wasserstoffatom oder den Benzylrest bedeutet, $R^{13}$ und $R^{14}$ jedoch nicht beide gleichzeitig für Wasserstoff stehen können, die Benzylgruppe durch katalytische Hydrierung in Gegenwart von Edelmetallkatalysatoren abspaltet und/ oder die Acylgruppe hydrolysiert und gegebenenfalls die erhaltenen basischen Verbindungen in üblicher Weise mit optisch aktiven Säuren zu Salzen umsetzt, aus den erhaltenen Salzen die optisch aktiven Basen in Freiheit setzt und die racemischen oder optisch aktiven Basen in an sich bekannter Weise in Salze mit physiologisch verträglichen Säuren überführt.

Eine Verbindung der Formel I, worin z.B. $R^1$, $R^2$ und/oder $R^3$ die Bedeutung von e) oder f) hat, kann man durch übliche Methoden wie z.B. Decarboxylierung (im Falle von $R^1$, $R^2$ und/oder $R^3$=COOH) oder Ammonolyse (im Falle von $R^1$, $R^2$ und/oder $R^3$=Alkoxycarbonyl) in eine solche umwandeln, worin $R^1$, $R^2$ und/oder $R^3$ Wasserstoff bzw. - $CONH_2$ bedeuten. Eine andere Variationsmöglichkeit ist z.B. die Umesterung entsprechender Verbindungen der Formel I.

Als durch nukleophile Substitution ersetzbare Gruppen W in Formel IV, X in Formel VI und Z in Formel VIII kommen z.B. in Betracht: Halogenatome sowie Schwefelsäure- oder Sulfonsäurereste.

Die Einführung des Aminrestes der Formel III nach der Verfahrensweise a) erfolgt beispielsweise durch Umsetzung der beiden Komponenten II und III, gegebenenfalls in Anwesenheit von organischen Lösungsmitteln wie Alkoholen, z.B. Methanol, Äthanol oder Isopropanol, aromatischen Kohlenwasserstoffen wie Benzol, Toluol oder Xylol, Äthern wir Diäthyläther, Dioxan, 1,2-Dimethoxyäthan, Tetrahydrofuran, aliphatischen oder alicyclischen Kohlenwasserstoffen wie Hexan, Heptan, Cyclohexan, Ketonen wie Aceton, Methyläthylketon, halogenierten Kohlenwasserstoffen wie Methylenchlorid, Chloroform, Carbonsäuredialkylamiden wie Dimethylformamid, Dimethylsulfoxid oder in Gegenwart von Wasser oder in Mischungen der genannten Lösungsmittel.

Als Reaktionsbedingungen kommen Temperaturen von Raumtemperatur bis zum Siedepunkt des Lösungsmittels und Normaldruck wie auch erhöhte Drucke infrage. Nach einer bevorzugten Ausführungsform läßt man die beiden Komponenten, in Alkohol gelöst, bei erhöhter Temperatur und Normaldruck miteinander reagieren.

Nach der unter b) bezeichneten Verfahrensweise wird als Ausgangsmaterial vorzugsweise ein 3-Thienyloxy-1-halogen-2-propanol der Formel IV eingesetzt. Anstelle der Halogenverbindung, vorzugsweise Chlor oder Brom kann auch ein entsprechender Ester der Schwefelsäure oder ein Ester von Sulfonsäuren verwendet werden. Zur Umsetzung mit einem Amin der Formel III arbeitet man in An- oder Abwesenheit von Lösungsmitteln und Reaktionsbedingungen wie unter Verfahrensweise a) angegeben. Zur Bindung der freiwerdenden Säure,

z.B. Halogenwasserstoff, kann man in Gegenwart von säurebindenden Mitteln, beispielsweise tert. Aminen wie Triäthylamin, Pyridin oder Alkali- oder Erdalkalihydroxiden, -carbonaten oder -hydrogencarbonaten arbeiten. Man kann auch die doppeltmolare Menge des eingesetzten Amins verwenden.

Die Ausgangsstoffe der Formel IV können beispielsweise durch Umsetzung eines 3-Hydroxythiophens der Formel VII mit Epichlorhydrin unter sauren Reaktionsbedingungen oder durch Aufspaltung eines Epoxids der Formel II mit einer Halogenwasserstoffsäure, Schwefelsäure oder einer Sulfonsäure erhalten werden. Sie können auch durch partielle Veresterung des Glycerinäthers eines Hydroxythiophens der Formel VIII erhalten werden.

Für die unter c) genannte Verfahrensweise wird das primäre Amin der Formel V eingesetzt; dieses kann auch als Salz vorliegen. Die Umsetzung mit einem reaktionsfähigen Ester der Formel VI erfolgt zweckmäßig unter den bei der Verfahrensweise b) genannten Bedingungen.

Das als Ausgangsstoff verwendete Amin der Formel V ist beispielsweise durch Umsetzung eines Epoxids der Formel II mit Ammoniak zugänglich. Es kann auch aus einem Halogenhydrin der Formel IV (W = Halogen) mit Ammoniak erhalten werden.

Die Verbindungen der Formel VI werden nach an sich literaturbekannten Verfahren hergestellt, beispielsweise durch Acylierungsreaktion des zugrunde liegenden Indols mit dem entsprechend substituierten Isobutancarbonsäurederivat und anschließende Reduktion der Carbonylfunktion zur Methylengruppe.

Bei der Herstellung der Verfahrensprodukte nach Verfahrensweise d) wird ein Thiophenderivat der Formel VII verwendet;

dieses kann auch in Form seines Alkalisalzes, wie Natrium- oder Kaliumsalz, eingesetzt werden. Als Reaktionspartner werden entsprechend substituierte 2-Propanolamine der Formel VIII verwendet, wobei Z einen bei einer nukleophilen Substitution leicht austauschbaren Rest darstellt. Hierbei kommen vor allem in Betracht Halogenide vorzugsweise Cl, Br, J aber auch entsprechende Schwefelsäure- oder Sulfonsäureester wie z.B. Mesylate oder Tosylate.

Die Umsetzung erfolgt vorteilhaft in Gegenwart eines säurebindenden Mittels, wie Alkalihydroxid. In alkalischem Medium kann das eingesetzte 2-Propanolamin VIII intermediär in das korrespondierende 1,2-Epoxypropan übergehen, welches mit dem Thiophenderivat reagiert. Die Umsetzung kann in An- oder Abwesenheit von Lösungsmitteln, wie Alkoholen, z.B. Methanol, Äthanol oder Isopropanol, aromatischen Kohlenwasserstoffen, wie Benzol, Toluol oder Xylol, Äthern wie Diäthyläther, Dioxan oder Tetrahydrofuran, Carbonsäureamiden wie Dimethylformamid bei Temperaturen im Bereich von Raumtemperatur bis zum Siedepunkt des verwendeten Lösungsmittels vorgenommen werden.

Die als Ausgangsstoffe verwendeten Verbindungen der Formel VIII (Z = Cl) sind beispielsweise durch Umsetzung eines Amins der Formel III mit Epichlorhydrin bei niedrigen Temperaturen zugänglich.

Gemäß Verfahren e) erhält man die Verbindungen der Formel I, indem man aus Verbindungen der Formel IX, in denen die Hydroxy- und/oder die sekundäre Aminogruppe durch die Reste $R^{13}$ bzw. $R^{14}$ geschützt ist (sind), diese Schutzgruppen abspaltet. Als Schutzgruppen kommen Acylreste und/oder der Benzylrest infrage. Die Abspaltung des Benzylrestes wird durch katalytische Hydrierung in Gegenwart von Edelmetallen, wie Palladium oder Platin durchgeführt.

0065295

Da der Acylrest bei der Reaktion gemäß Verfahren e) abgespalten wird, hat seine chemische Konstitution keinen Einfluß auf den Charakter des Endproduktes und kann deshalb in weiten Grenzen variiert werden. Bevorzugt werden jedoch Acylverbindungen, wobei der Acylrest ein niederer aliphatischer Acylrest wie der Acetyl- oder Propionylrest ist. Die Abspaltung erfolgt hydrolytisch entweder in saurem oder alkalischem Medium.

Hat man Acyl- und Benzylreste als $R^{13}$ und $R^{14}$ nebeneinander, so kann man nacheinander in der beschriebenen Weise die Gruppen abspalten.

Die Herstellung der entsprechenden Benzyl- und/oder Acylverbindungen der Formel IX kann nach einer der oben beschriebenen Methoden durchgeführt werden, wobei die entsprechenden acylierten und/oder benzylierten Ausgangsstoffe verwendet werden. Sollen Ausgangsstoffe der Formel IX hergestellt werden, in denen $R^{13}$ einen Acylrest bedeutet, so kann beispielsweise die Verbindung der Formel III acyliert werden, worauf anschließend die entsprechenden Acylverbindungen nach der Methode b) zu Verbindungen der Formel IX umgesetzt werden. Entsprechendes gilt für Verbindungen, in denen $R^{13}$ den Benzylrest bedeuten, wobei anstelle der Acylierung die Benzylierung der entsprechenden Hydroxyverbindung tritt. Will man von Verbindungen der Formel IX ausgehen, in denen $R^{14}$ den Benzylrest bedeutet, so kann man nach den Verfahrenseisen a), b), c), oder d) anstelle der Amine die entsprechenden N-Benzylverbindungen einsetzen.

Die Verfahrensprodukte können als freie Basen oder in Form ihrer Salze anfallen, und falls erforderlich, durch die üblichen Aufarbeitungsmethoden, beispielsweise durch Umkristallisieren oder gegebenenfalls Überführen in die freie

Base und anschließende Salzbildung oder gegebenenfalls durch Säulenchromatographie gereinigt werden.

Es kann mitunter von Vorteil sein, die Herstellung der Ausgangsstoffe direkt mit der weiteren Umsetzung zu kombinieren, d.h. die Zwischenprodukte nicht gesondert zu isolieren.

Die Verfahrensprodukte können gegebenenfalls in die Salze physiologisch verträglicher organischer oder anorganischer Säuren übergeführt werden.

Als organische Säuren seien beispielsweise genannt: Essigsäure, Propionsäure, Milchsäure, Malonsäure, Bernsteinsäure, Maleinsäure, Fumarsäure, Zitronensäure, Äpfelsäure Weinsäure, Benzoesäure, Salicylsäure, Oxäthansulfonsäure, Acetursäure (Acetylaminoessigsäure), Äthylendiamintetraessigsäure, Embonsäure (1,1'-Methylen-bis-(2-hydroxy-3-naphthoesäure)) sowie saure Gruppen enthaltenden synthetische Harze.

Als anorganische Säuren kommen beispielsweise in Betracht: Halogenwasserstoffsäure, wie Chlorwasserstoffsäure oder Bromwasserstoffsäure, Schwefelsäure, Phosphorsäure und Amidosulfonsäure.

Da die erfindungsgemäßen Verbindungen ein Chiralitätszentrum enthalten, können die racemischen Verbindungen mit optisch aktiven Säuren in die Antipoden zerlegt werden. Die Erfindung betrifft daher die racemischen Verbindungen sowie die optisch aktiven Verbindungen, die der Einfachheit halber alle durch die Formel I wiedergegeben werden.

Als Säuren, wie sie für die Herstellung optisch aktiver Salze nach der Erfindung infrage kommen, seien beispielsweise genannt:

(+)- und (-)-Weinsäure, (+)- und (-)-Dibenzoylweinsäure,

(+)- und (-)-Ditoluylweinsäure, (+)- und (-)-Mandelsäure, (+)- und (-)-Camphersäure, (+)-Campher-ß-sulfonsäure, (+)- und (-)-α-Bromcampher-π-sulfonsäure und N-(-Nitrobenzoyl)-(+)-glutaminsäure.

Die Herstellung der optisch aktiven Salze kann in Wasser, wasserhaltigen oder wasserfreien organischen Lösungsmitteln erfolgen. Vorteilhaft erweist sich die Verwendung von Alkoholen oder von Estern organischer Säuren.

Zur Herstellung von optisch aktiven Verbindungen setzt man das Racemat der Base in einem Lösungsmittel, vorzugsweise in molaren Mengenverhältnissen, mit einer optisch aktiven Säure um und isoliert das optisch aktive Salz der Verbindungen der Formel I. Man kann auch in bestimmten Fällen nur ein halbes Äquivalent der optisch aktiven Säure verwenden, um den einen optisch aktiven Antipoden aus dem Racemat zu entfernen, wie man ebenso auch überschüssige Mengen an optisch aktiver Säure einsetzen kann.

Je nach Art der optisch aktiven Säure kann der gewünschte Antipode in Form seines diastereomeren Salzes entweder direkt oder aus der Mutterlauge des ersten Kristallisats erhalten werden und gegebenenfalls durch mehrmalige Kristallisation des diastereomeren Salzes gereinigt werden. Man kann anschließend das Salz in die Base überführen und die wieder in Freiheit gesetzte optisch aktive Base in ein Salz einer physiologisch verträglichen organischen oder anorganischen Säuren überführen.

Man kann zu den optisch aktiven Antipoden der Verfahrensprodukte auch gelangen, wenn man eine geeignete Vor-(Zwischen-)stufe des erfindungsgemäßen Verfahrens, beispielsweise ein Amin der Formel V nach Verfahrensweise c), eine Verbindung der Formel VIII nach Verfahrensweise d) oder eine Verbindung der Formel IX nach Verfahrensweise e) mit einer optisch aktiven Säure in die Komponenten zerlegt und

mit den so erhaltenen optisch aktiven Antipoden die Verfahrensprodukte nach den entsprechenden Verfahrensweisen synthetisiert.

Die erfindungsgemäßen Verbindungen haben wertvolle pharmakologische Eigenschaften und sind daher insbesondere als therapeutische Mittel für die Behandlung von Herz- und Kreislauferkrankungen geeignet. Sie weisen u.a. betasympatholytische, antihypertensive, antiarrhythmische, vasodilatierende, antianginöse und antithrombotische Eigenschaften auf.

Die antihypertensive Wirksamkeit der erfindungsgemäßen Verbindungen kann in verschiedenen pharmakolgischen Tests nachgewiesen werden. So wirkt die Verbindung gemäß Beispiel 2 sowohl am narkotisierten Hund als auch am wachen Hund ähnlich stark und anhaltend blutdrucksenkend wie das bekannte Bucindolol(2-<2-Hydroxy-3-<[2-(3-indolyl)-1,1-dimethyläthyl] amino>-propoxy>benzonitril), ist aber - wie die nachfolgende Tabelle zeigt - deutlich ungiftiger als die Vergleichsverbindung.

| Akute Toxizität | Verb. gem. Beisp. 2 | Bucindolol |
|---|---|---|
| $LD_{50}$ Maus i.v. | 74 mg/kg | 9,6 mg/kg |
| $LD_{50}$ Ratte i.v. | 86 mg/kg | 20,2 mg/kg |

Die neuen Verbindungen können entweder allein oder mit physiologisch verträglichen Hilfs- oder Trägerstoffen vermischt angewandt werden. Für eine orale Anwendungsform werden die aktiven Verbindungen mit den dafür üblichen Substanzen vermischt und durch übliche Methoden in geeignete Darreichungsformen gebracht, wie Tabletten, Steckkapseln, wäßrige, alkoholische oder ölige Suspensionen oder wäßrige

alkoholische oder ölige Lösungen. Als inerte Träger können z.B. Magnesiumcarbonat, Milchzucker oder Maisstärke verwendet werden. Dabei kann die Zubereitung sowohl als Trocken- oder Feuchtgranulat erfolgen. Als ölige Trägerstoffe oder Lösungsmittel kommen beispielsweise besonders pflanzliche und tierische Öle in Betracht, wie z.B. Sonnenblumenöl oder Lebertran.

Eine besondere Anwendungsform liegt in der intravenösen Applikation. Zu diesem Zweck werden die aktiven Verbindungen oder deren physiologisch verträglichen Salze mit den dafür üblichen Substanzen in Lösung gebracht.

Die erfindungsgemäßen Verbindungen werden zweckmäßig in täglichen Dosierungen von 1 bis 500 mg oral bzw. 0,5 bis 50 mg intravenös verabreicht. Die Verbindung gemäß Beispiel 2 wird vorzugsweise in täglichen Dosierungen von 5 bis 180 mg oral appliziert, eine Tablette oder ein Dragee enthält vorzugsweise 5 bis 90 mg Wirkstoff.

Als Lösungsmittel der entsprechenden physiologisch verträglichen Salze der aktiven Verbindungen für eine intravenöse Applikation kommen z.B. in Frage: physiologische Kochsalzlösungen oder Alkohol, wie z.B. Äthanol, Propandiol oder Glycerin, daneben auch Zuckerlösungen wie z.B. Glukose- oder Mannitlösungen, oder auch eine Mischung aus den verschiedenen genannten Lösungsmitteln.

Beispiel 1

3-<3-/1,1-Dimethyl- 2-(indol-3-yl)äthylamino/-2-hydroxy-propoxy>-thiophen-2-carbonsäuremethylester

Man rührt 1,88 g (= 0,01 Mol) 1,1-Dimethyl-2-(indol-3-yl)-äthylamin, gelöst in 100 ml Äthanol, bei Siedetemperatur, tropft 21,4 g (= 0,01 Mol) 3-(2,3-Epoxypropoxy)thiophen-2-carbonsäuremethylester ($Kp_{0,1}$: 154-156°C; - dargestellt

durch Umsetzung von 3-Hydroxythiophen-2-carbonsäuremethylester mit Epichlorhydrin in Gegenwart von Alkali), gelöst in 50 ml Äthanol, hinzu und rührt anschließend noch 5 Stunden bei Siedetemperatur nach. Nach dem Einengen der Reaktionslösung im Vakuum isoliert man das Reaktionsprodukt durch Säulenchromatographie an Kieselgel (Laufmittel: Methylenchlorid, Methanol, konz. Ammoniak; 90 : 9 : 1). Die freie Base bildet mit der berechneten Menge Oxalsäure in Äthanol das kristalline 3-<3-/‾1,1-Dimethyl-2-(indol-3-yl)äthylamino_7-2-hydroxypropoxy>-thiophen-2-carbonsäuremethylester-hydrogenoxalat, Schmp.: 180-183°C (Zers.).

Beispiel 2

3-<3-/‾1,1-Dimethyl-2-(indol-3-yl)äthylamino_7-2-hydroxypropoxy>-thiophen-2-carbonsäureamid

Man rührt 7,99 g (= 0,045 Mol) 1,1-Dimethyl-2-(indol-3-yl)-äthylamin, gelöst in 300 ml Äthanol, bei Siedetemperatur, tropft 8,97 g (= 0,045 Mol) 3-(2,3-Epoxypropoxy)thiophen-2-carbonsäureamid (F.: 120-122°C), gelöst in 400 ml Äthanol, hinzu und rührt anschließend noch 3 Stunden bei Siedetemperatur nach. Nach dem Einengen der Reaktionslösung im Vakuum isoliert man das Reaktionsprodukt durch Säulenchromatographie an Kieselgel (Laufmittel: Methanol) und erhält so 15,1 g kristallines 3-<3-/‾1,1-Dimethyl-2-(indol-3-yl)äthylamino_7-2-hydroxypropoxy -thiophen-2-carbonsäureamid /‾Schmp.: ca. 94-98°C (Zers.)_7. Die Base wird mit äthanolischer Salzsäure in das kristalline Hydrochlorid überführt, Schmp.: 155-158°C.

Beispiel 3

3-<3-/‾1,1-Dimethyl-2-(indol-3-yl)äthylamino_7-2-hydroxypropoxy>-thiophen-2-carbonsäuremorpholid

1,01 g (= 0,004 Mol) 3-(2,3-Epoxypropoxy)thiophen-2-carbonsäuremorpholid und 0,75 g (= 0,004 Mol) 1,1-Dimethyl-2-

(indol-3-yl)äthylamin werden 1 Stunde bei 130°C gerührt. Man läßt erkalten, die erkaltete Schmelze wird mit Methylenchlorid/Wasser behandelt, die Methylenchloridphase abgetrennt, getrocknet und im Vakuum eingeengt. Man isoliert das 3-<3-[1,1-Dimethyl-2-(indol-3-yl)äthylamino]-2-hydroxypropoxy>-thiophen-2-carbonsäuremorpholid durch Säulenchromatographie an Kieselgel (Laufmittel: Methanol, Äthylacetat; 1 : 1). Es bildet mit der berechneten Menge Oxalsäure in Äthanol das kristalline Hydrogenoxalat, Schmp.: 146-148°C (Zers.).

Beispiel 4

3-<3-[1,1-Dimethyl-2-(indol-3-yl)äthylamino]-2-hydroxypropoxy>-thiophen-2-carbonsäurepiperidid

Unter analogen Reaktionsbedingungen wie in Beispiel 3 beschrieben erhält man aus 3-(2,3-Epoxypropoxy)thiophen-2-carbonsäurepiperidid und 1,1-Dimethyl-2-indol-3-yl)äthylamin das 3-<3-[1,1-Dimethyl-2-(indol-3-yl)äthylamino]-2-hydroxypropoxy>thiophen-2-carbonsäurepiperidid und das Hydrogenoxalat, Schmp.: 181-183°C (Zers.).

Beispiel 5

3-<3-[1,1-Dimethyl-2-(indol-3-yl)äthylamino]-2-hydroxypropoxy>-thiophen-2-carbonsäure-(4-methoxyanilid)

Unter analogen Reaktionsbedingungen wie in Beispiel 3 beschrieben erhält man aus 3-(2,3-Epoxypropoxy)thiophen-2-carbonsäure-(4-methoxyanilid) und 1,1-Dimethyl-2-indol-3-yl)äthylamin das 3-<3-[1,1-Dimethyl-2-(indol-3-yl)äthylamino]-2-hydroxypropoxy>thiophen-2-carbonsäure-(4-methoxyanilid) und das Hydrogenoxalat vom Schmp.: 205-207°C (Zers.).

Beispiel 6

3-<3-/1,1-Dimethyl-2-(indol-3-yl)äthylamino/-2-hydroxy-propoxy>-thiophen-2-carbonsäurenitril

1,81 g (= 0,01 Mol) 3-(2,3-Epoxypropoxy)thiophen-2-carbonsäurenitril und 1,86 g (= 0,01 Mol) 1,1-Dimethyl-2-indol-3-yl)äthylamin werden 1 Stunde bei ca. 130°C gerührt. Man läßt erkalten, die erkaltete Schmelze wird mit Methylenchlorid/Wasser behandelt, die Methylenchloridphase abgetrennt, getrocknet und eingeengt. Das als Rückstand verbleibende 3-<3-/1,1-Dimethyl-2-(indol-3-yl)äthylamino/-2-hydroxypropoxy>thiophen-2-carbonsäurenitril bildet mit der berechneten Menge Oxalsäure ein kristallines Hydrogenoxalat, Schmp.: 160-163°C (Zers.).

Beispiel 7

3-<3-/1,1-Dimethyl-2-(indol-3-yl)äthylamino/-2-hydroxy-propoxy>-thiopohen-2-carbonsäureamid

Man rührt 1,88 g 1,1-Dimethyl-2-(indol-3-yl)äthylamin (0,01 Mol), gelöst in 100 ml Äthanol, bei Siedetemperatur, fügt 0,92 g Natriumhydrogencarbonat hinzu und tropft 2,35 g 3-(3-Chlor-2-hydroxypropoxy)thiophen-2-carbonsäureamid (0,01 Mol), gelöst in 50 ml Äthanol, langsam zu. Man rührt anschließend noch 24 Stdn. bei Siedetemperatur nach, filtriert dann die Reaktionslösung und engt i. Vak. zur Trockne ein. Aus dem Rückstand isoliert man durch Chromatographie an Kieselgel (Laufmittel: Methanol) 3-<3-/1,1-Dimethyl-2-(indol-3-yl)äthylamino/-2-hydroxypropoxy>-thiophen-2-carbonsäureamid, dessen Hydrochlorid mit dem nach Beispiel 2 erhaltenen Produkt identisch ist.

Beispiel 8

3-<3-[1,1-Dimethyl-2-(indol-3-yl)äthylamino]-2-hydroxy-propoxy>-thiophen-2-carbonsäureamid

2,81 g N-(3-Chlor-2-hydroxypropoxy)-1,1-dimethyl-2-(indol-3-yl)-äthylamin [0,01 Mol; dargestellt nach THL 23, (1967), 2130 durch Umsetzung von 1,1-Dimethyl-2-(indol-3-yl)äthylamin mit Epichlorhydrin], gelöst in 100 ml Äthanol, tropft man bei Raumtemperatur langsam zu einer intensiv gerührten Mischung von 3-Hydroxythiophen-2-carbonsäureamid (0,01 Mol) und 1,26 g feingepulvertem Natriumhydrogencarbonat in 100 ml Äthanol und rührt die Reaktionsmischung 24 Stdn. bei Siedetemperatur nach. Die filtrierte Mischung wird i.Vak. zur Trockne eingeengt. Nach Chromatographie an Kieselgel (Laufmittel: Methanol) isoliert man das 3-<3-[1,1-Dimethyl-2-(indol-3-yl)äthylamino]-2-hydroxypropoxy>-thiophen-2-carbonsäureamid, dessen Hydrochlorid mit dem nach Beispiel 2 erhaltenen Produkt identisch ist.

Beispiel 9

3-<3-[1,1-Dimethyl-2-(indol-3-yl)äthylamino]-2-hydroxy-propoxy>-thiophen-2-carbonsäureamid

Man löst 4,78 g 3-<3-[N-Benzyl-1,1-dimethyl-2-(indol-3-yl) äthylamino]-2-hydroxypropoxy>-thiophen-2-carbonsäureamid [0,01 Mol; dargestellt durch Umsetzung von N-Benzyl-1,1-dimethyl-2-(indol-3-yl)äthylamin mit 3-(2,3-Epoxypropoxy) thiophen-2-carbonsäureamid] in 250 ml Äthanol und hydriert nach Zugabe von 2 g Pd/C-Katalysator (10-prozentig) bei 50 atü und $80^{\circ}$ bis zur Aufnahme der gerechneten Menge Wasserstoff. Nach dem Abfiltrieren des Katalysator zieht man das Lösungsmittel i.Vak. ab und versetzt den Rückstand mit äthanolischer HCl. Das erhaltene 3-<3-[1,1-Dimethyl-2-(indol-3-yl)äthylamino]-2-hydroxypropoxy>-thiophen-2-carbonsäureamid-hydrochlorid ist mit dem nach Beispiel 2 erhaltenen Produkt identisch.

## Beispiel 10

3-<3-[1,1-Dimethyl-2-(indol-3-yl)äthylamino]-2-hydroxypropoxy>-thionaphthen

1,88 g Dimethyl-2-(indol-3-yl)äthylamin (0,01 Mol), gelöst in 50 ml Äthanol, rührt man bei Siedetemperatur. Man tropft 2,05 g 3-(2,3-Epoxypropoxy)thionaphten/ 0,01 Mol; dargestellt nach Heterocyl. Chem. 17, (1980), 937-940 /, gelöst in 50 ml Äthanol, langsam zu und rührt die Reaktionsmischung 24 Stdn. bei dieser Temperatur nach. Man zieht anschließend das Lösungsmittel ab, chromatographiert den Rückstand an Kieselgel (Laufmittel: Essigester/Methanol; 1:1) und erhält 3-<3-[1,1-Dimethyl-2-(indol-3-yl)äthylamino]-2-hydroxypropoxy>-thionaphthen (Schmp.: 104°).

Die gleiche Verbindung erhält man durch thermische Decarboxylierung von 3-<3-[1,1-Dimethyl-2-(indol-3-yl)äthylamino]-2-hydroxypropoxy>-thionaphthen-2-carbonsäure (Schmp.: 205° (Zers.), welche beispielsweise durch alkalische Verseifung der Reaktionsprodukte aus 3-(2,3-Epoxypropoxy)-thionaphthen-2-carbonsäuremethylester (vgl. Heteroapl. Chem. 17 (1980), 937-940) und 1,1-Dimethyl-2-(indol-3-yl)äthylamin zugänglich ist.

## Beispiel 11

3-<3-[1,1-Dimethyl-2-(indol-3-yl)äthylamino]-2-hydroxypropoxy>-thionaphthen-2-carbonsäureamid

Man rührt 0,4 g 1,1-Dimethyl-2-(indol-3-yl)äthylamin (0,002 Mol), gelöst in 25 ml Äthanol, bei Siedetemperatur, tropft 0,5 g 3-(2,3-Epoxypropoxy)thionaphthen-2-carbonsäureamid (0,002 Mol), gelöst in 25 ml Äthanol, bei dieser Temperatur zu und rührt die Reaktionsmischung 10 Stdn. bei Siedetemperatur nach. Man zieht dann das Lösungsmittel i.Vak. ab und chromatographiert den Rückstand an Kieselgel (Laufmittel: Methanol) und erhält 3-<3-[1,1-Dimethyl-2-(indol-3-yl)äthylamino]-2-hydroxypropoxy>-thionaphthen-2-carbonsäureamid (Schmp.: 122°; Zers.).

Beispiel 12

3-⟨3-[1,1-Dimethyl-2-(indol-3-yl)äthylamino]-2-hydroxypropoxy⟩-thiophen-2-carbonsäuremethylamid

Aus 1,1,-Dimethyl-2-(indol-3-yl)äthylamin und 3-(2,3-Epoxypropoxy)thiophen-2-carbonsäuremethylamid entsprechend Beispiel 2.
F.: 164° (Hydrochlorid)

Beiepiel 13

3-⟨3-[1,1-Dimethyl-2-(indol-3-yl)äthylamino]-2-hydroxypropoxy⟩-thiophen-2-carbonsäure-n-propylamid

Aus 1,1-Dimethyl-2-(indol-3-yl)äthylamin und 3-(2,3-Epoxypropoxy)thiophen-2-carbonsäure-n-propylamid entsprechend Beispiel 2.
F.: 125° (Hydrogenoxalat)

Beispiel 14

3-⟨3-[1,1-Dimethyl-2-(indol-3-yl)äthylamino]-2-hydroxypropoxy⟩-thiophen-2-carbonsäuredimethylamid

Aus 1,1-Dimethyl-2-(indol-3-yl)äthylamin und 3-(2,3-Epoxypropoxy)thiophen-2-carbonsäuredimethylamid entsprechend Beispiel 2.
F.: 155° (Hydrogenoxalat)

Beispiel 15

4-⟨3-[1,1-Dimethyl-2-(indol-3-yl)äthylamino]-2-hydroxypropoxy -thiophen-2-carbonsäuremethylester

Aus 1,1-Dimethyl-2-(indol-3-yl)äthylamin und 4-(2,3-Epoxypropoxy)thiophen-2-carbonsäuremethylester entsprechend Beispiel 2.
F.: 166° (Hydrogenoxalat)

Beispiel 16

4-<3-/ 1,1-Dimethyl-2-indol-3-yl)äthylamino 7-2-hydroxy-propoxy>-thiophen-2-carbonsäureamid

Aus 1,1-Dimethyl-2-(indol-3-yl)äthylamin und 4-(2,3-Epoxy-propoxy)thiophen-2-carbonsäureamid entsprechend Beispiel 2.
F.: 118° (Zers.; Hydrochlorid)
Das Reaktionsprodukt kann auch erhalten werden durch Ammonolyse von 4-<3-/ 1,1-Dimethyl-2-(indol-3-yl)äthyl-amino 7-2-hydroxypropoxy>-thiophen-2-carbonsäuremethylester (Herst. vgl. Beispiel 15).

Beispiel 17

5-Acetyl-4-<3-/ 1,1-dimethyl-2-indol-3-yl)äthylamino 7-2-hydroxypropoxy>-thiophen-2-carbonsäureäthylester

Aus 1,1-Dimethyl-2-(indol-3-yl)äthylamin und 5-Acetyl-4-(2,3-Epoxypropoxy)thiophen-2-carbonsäureäthylester entsprechend Beispiel 2.
F.: 142°

Beispiel 18

3-<3-/ 1,1-Dimethyl-2-(5-bromindol-3-yl)äthylamino 7-2-hydroxypropoxy>thiophen-2-carbonsäureamid

Aus 1,1-Dimethyl-2-(5-bromindol-3-yl)äthylamin und 3-(2,3-Epoxypropoxy)thiophen-2-carbonsäureamid entsprechend Beispiel 2.
F.: 257-258° (Hydrochlorid)

Beispiel 19

3-<3-/ 1,1-Dimethyl-2-(7-methoxyindol-3-yl)äthylamino 7-2-hydroxypropoxy>thiophen-2-carbonsäureamid

Aus 1,1-Dimethyl-2-(7-methoxyindol-3-yl)äthylamin und 3-(2,3-Epoxypropoxy)thiophen-2-carbonsäureamid entsprechend Beispiel 2.
F.: 225-226° (Hydrochlorid)

Beispiel 20

3-<3-[1,1-Dimethyl-2-(5-methoxyindol-3-yl)äthylamino]-2-hydroxypropoxy>thiophen-2-carbonsäureamid

Aus 1,1-Dimethyl-2-(5-methoxyindol-3-yl)äthylamin und 3-(2,3-Epoxypropoxy)thiophen-2-carbonsäureamid entsprechend Beispiel 2.
F.: 208-210° (Hydrochlorid)

Beispiel 21

3-<3-[1,1-Dimethyl-2-(2,5-dimethylindol-3-yl)äthylamino]-2-hydroxypropoxy>thiophen-2-carbonsäureamid

Aus 1,1-Dimethyl-2-(2,5-dimethylindol-3-yl)äthylamin und 3-(2,3-Epoxypropoxy)thiophen-2-carbonsäureamid entsprechend Beispiel 2.
F.: 265-267° (Hydrochlorid)

Beispiel 22

3-<3-[1,1-Dimethyl-2-(2-methylindol-3-yl)äthylamino]-2-hydroxypropoxy>thiophen-2-carbonsäureamid

Aus 1,1-Dimthyl-2-(2-methylindol-3-yl)äthylamin und 3-(2,3-Epoxypropoxy)thiophen-2-carbonsäureamid entsprechend Beispiel 2.
F.: 217° (Hydrochlorid)

Beispiel 23

3-<3-[1,1-Dimethyl-2-(2-carbamoylindol-3-yl)äthylamino]-2-hydroxypropoxy>thiophen-2-carbonsäureamid

Aus 1,1-Dimethyl-2-(2-carbamoylindol-3-yl)äthylamin und 3-(2,3-Epoxypropoxy)thiophen-2-carbonsäureamid entsprechend Beispiel 2.
F.: 236-238° (Hydrochlorid)

Beispiel 24

3-<3-[1,1-Dimethyl-2-(2-carbamoyl-1-methylindol-3-yl)äthylamino]-2-hydroxypropoxy>thiophen-2-carbonsäureamid

Aus 1,1-Dimethyl-2-(2-carbamoyl-1-methylindol-3-yl)äthyl-amin und 3-(2,3-Epoxypropoxy)thiophen-2-carbonsäureamid entsprechend Beispiel 2.
F.: 204-206° (Hydrogenoxalat)

Patentansprüche:

1. Racemische und optisch aktive substituierte Tryptaminderivate von Thienyloxypropanolaminen der Formel I

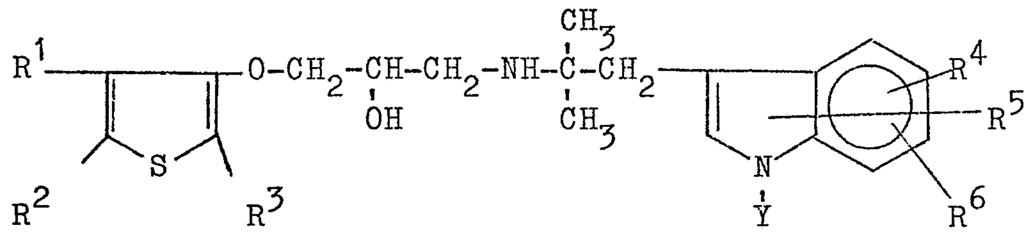

I

worin bedeuten:

$R^1$, $R^2$ und $R^3$

a) Wasserstoff, Halogen, Alkyl mit 1 bis 6 C-Atomen, Alkoxy mit 1 bis 4 C-Atomen, Alkoxymethyl mit 1 bis 4 C-Atomen im Alkoxylteil, Hydroxymethyl,

b) Phenyl, gegebenenfalls mono- di- oder trisubstituiert mit Halogen, $C_1$-$C_6$-Alkyl oder $C_1$-$C_4$-Alkoxy, oder Phenylalkyl mit 1 bis 4 C-Atomen im Alkylteil,

c) Cycloalkyl mit 3 bis 8 C-Atomen,

d) Cyano, Nitro, Amino,

e) Carboxyl, Alkoxycarbonyl mit 1 bis 4 C-Atomen im Alkoxylteil,

f) Carbonamido der Formel 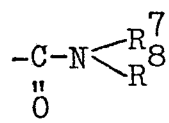

worin $R^7$ und $R^8$ gleich oder verschieden sind und Wasserstoff, Alkyl mit 1 bis 6 C-Atomen, oder Phenyl, gegebenenfalls substituiert mit $C_1$-$C_4$-Alkyl, $C_1$-$C_4$-Alkoxy oder Halogen, bedeuten oder

worin $R^7$ und $R^8$ Alkylreste sind, die gemeinsam mit dem Stickstoffatom einen 5- bis 7-gliedrigen Ring bilden, wobei eine $-CH_2$-Gruppe durch Sauerstoff, Schwefel oder die $NR^9$-Gruppe ersetzt sein kann, worin $R^9$ Wasserstoff, $C_1$-$C_4$-Alkyl, Phenyl oder Phenylalkyl mit 1 bis 2 C-Atomen im Alkylteil bedeutet,

g) $R^1$ und $R^2$ zusammen mit dem Thiophenring ein kondensiertes heteroaromatisches Ringsystem mit insgesamt 8 bis 9 Ringgliedern und 1 bis 2 Schwefelatomen, wobei $R^3$ die unter a) bis f) genannten Bedeutungen besitzt,

$R^4$, $R^5$ und $R^6$

a) Wasserstoff, Halogen, Alkyl mit 1 bis 6 C-Atomen, Alkoxy mit 1 bis 4 C-Atomen, Hydroxy,

b) Phenyl, gegebenenfalls mono- oder disubstituiert mit $C_1$-$C_4$-Alkyl, $C_1$-$C_4$-Alkoxy oder Halogen,

c) Nitro, Amino,

d) Carboxyl, Alkoxycarbonyl mit 1 bis 4 C-Atomen im Alkoxylteil oder Carbonamido der Formel 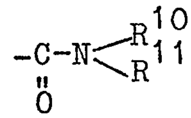

worin $R^{10}$ und $R^{11}$ gleich oder verschieden sind und Wasserstoff oder Alkyl mit 1 bis 6 C-Atomen bedeuten, wobei die Alkylreste gemeinsam mit dem Stickstoffatom einen 5- bis 7-gliedrigen Ring bilden können, worin eine $-CH_2$-Gruppe durch Sauerstoff, Stickstoff oder die $NR^{12}$-Gruppe, worin $R^{12}$ Wasserstoff, $C_1$-$C_4$-Alkyl, Phenyl oder Phenylalkyl mit 1 bis 2 C-Atomen im Alkylteil bedeutet, ersetzt sein kann,

Y Wasserstoff, $C_1$-$C_4$-Alkyl oder Phenyl

und deren physiologisch verträgliche Salze mit Säuren.

2. Verfahren zur Herstellung von Verbindungen gemäß Anspruch 1, dadurch gekennzeichnet, daß man

a) ein Epoxid der Formel II,

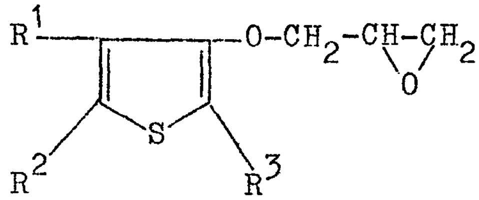

II

worin $R^1$, $R^2$ und $R^3$ die obige Bedeutung haben, mit einem Amin der Formel III,

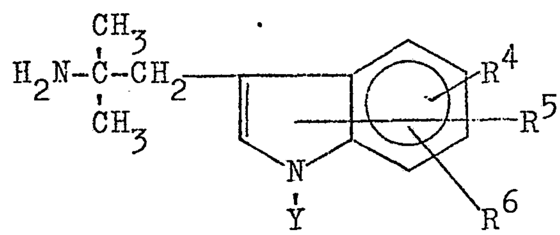

III

worin $R^4$, $R^5$, $R^6$ und Y die obige Bedeutung haben, umsetzt oder

b) eine Verbindung der Formel IV,

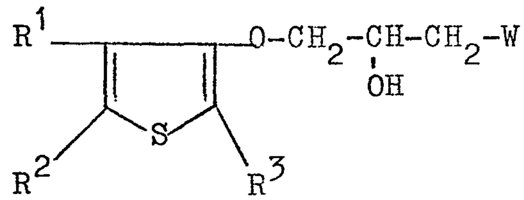

IV

worin $R^1$, $R^2$ und $R^3$ die obige Bedeutung haben und W eine durch nukleophile Substitution ersetzbare Gruppe darstellt, mit einem Amin der Formel III, worin $R^4$, $R^5$, $R^6$ und Y die obige Bedeutung haben, umsetzt, oder

c) ein Amin der Formel V,

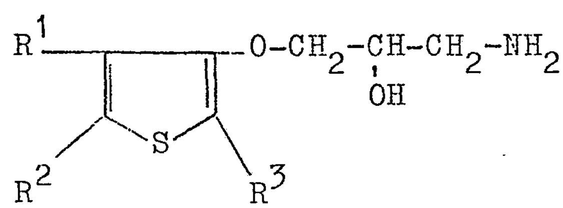

V

worin $R^1$, $R^2$ und $R^3$ die obige Bedeutung haben, mit einer Verbindung der Formel VI,

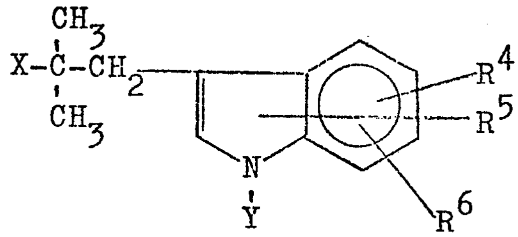

VI

worin $R^4$, $R^5$, $R^6$ und Ydie obige Bedeutung haben und X eine durch nukleophile Substitution ersetzbare Gruppe darstellt, umsetzt, oder

d) ein Thiophenderivat der Formel VII,

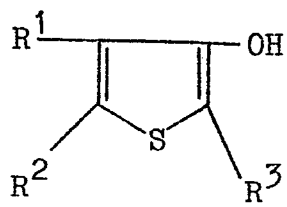

VII

 

worin $R^1$, $R^2$ und $R^3$ die obige Bedeutung haben, mit einer Verbindung der Formel VIII,

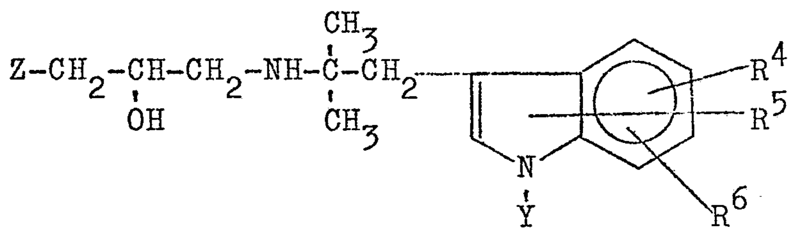

VIII

worin $R^4$, $R^5$, $R^6$ und Y die zu Formel I angegebene Bedeutung haben und Z eine durch nukleophile Substitution ersetzbare Gruppe darstellt, in Gegenwart eines säurebindenden Mittels umsetzt, oder

e) in einer Verbindung der Formel IX

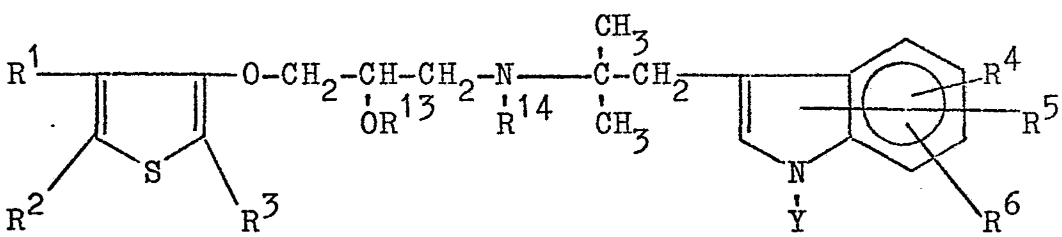

IX

worin $R^1$, $R^2$, $R^3$, $R^4$, $R^5$, $R^6$ und Y die obige Bedeutung haben, $R^{13}$ ein Wasserstoffatom, einen Acylrest oder den Benzylrest und $R^{14}$ ein Wasserstoffatom oder den Benzylrest bedeutet, $R^{13}$ und $R^{14}$ jedoch nicht beide gleichzeitig für Wasserstoff stehen können, die Benzylgruppe durch katalytische Hydrierung in Gegenwart von Edelmetallkatalysatoren abspaltet und/oder die Acylgruppe hydrolysiert und

gegebenenfalls die erhaltenen basischen Verbindungen in üblicher Weise mit optisch aktiven Säuren zu Salzen umsetzt, aus den erhaltenen Salzen die optisch aktiven Basen in Freiheit setzt und die racemischen oder optisch

aktiven Basen in an sich bekannter Weise in Salze mit physiologisch verträglichen Säuren überführt.

3. Pharmazeutische Präparate auf Basis einer Verbindung gemäß Anspruch 1.

4. Verfahren zur Herstellung eines pharmazeutischen Präparates gemäß Anspruch 3, dadurch gekennzeichnet, daß man eine Verbindung gemäß Anspruch 1, gegebenenfalls unter Zusatz von üblichen Hilfsstoffen, in eine pharmazeutisch übliche Darreichungsform überführt.

5. Verwendung von Verbindungen gemäß Anspruch 1 bei der Behandlung von Herz-Kreislauferkrankungen.

Patentanspruch für Österreich

Verfahren zur Herstellung von racemischen und optisch aktiven substituierten Tryptaminderivaten von Thienyloxypropanolaminen der Formel I

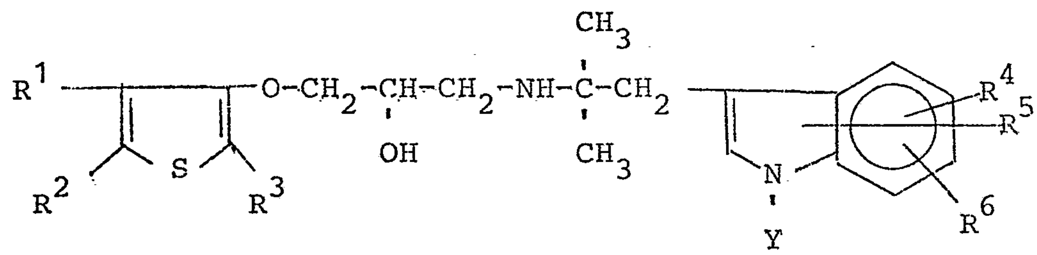

I

worin beudeten:

$R^1$, $R^2$ und $R^3$

a) Wasserstoff, Halogen, Alkyl mit 1 bis 6 C-Atomen, Alkoxy mit 1 bis 4 C-Atomen, Alkoxymethyl mit 1 bis 4 C-Atomen im Alkoxyteil, Hydroxymethyl,

b) Phenyl, gegebenenfalls mono-, di- oder trisubstituiert mit Halogen, $C_1$-$C_6$-Alkyl oder $C_1$-$C_4$-Alkoxy, oder Phenylalkyl mit 1 bis 4 C-Atomen im Alkylteil,

c) Cycloalkyl mit 3 bis 8 C-Atomen,

d) Cyano, Nitro, Amino,

e) Carboxyl, Alkoxycarbonyl mit 1 bis 4 C-Atomen im Alkoxyteil,

f) Carbonamido der Formel 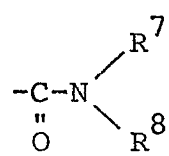

worin $R^7$ und $R^8$ gleich oder verschieden sind und Wasserstoff, Alkyl mit 1 bis 6 C-Atomen, oder Phenyl, gegebenenfalls substituiert mit $C_1$-$C_4$-Alkyl, $C_1$-$C_4$-Alkoxy oder Halogen, bedeuten oder worin $R^7$ und $R^8$ Alkylreste sind, die gemeinsam mit dem Stickstoffatom einen 5- bis 7-gliedrigen Ring bilden, wobei eine $-CH_2$-Gruppe durch Sauerstoff, Schwefel oder die $NR^9$-Gruppe ersetzt sein kann, worin $R^9$

Wasserstoff, $C_1$-$C_4$-Alkyl, Phenyl oder Phenylalkyl mit 1 bis 2 C-Atomen im Alkylteil bedeutet,

g) $R^1$ und $R^2$ zusammen mit dem Thiophenring ein kondensiertes heteroaromatisches Ringsystem mit insgesamt 8 bis 9 Ringgliedern und 1 bis 2 Schwefelatomen, wobei $R^3$ die unter a) bis f) genannten Bedeutungen besitzt,

$R^4$, $R^5$ und $R^6$

a) Wasserstoff, Halogen, Alkyl mit 1 bis 6 C-Atomen, Alkoxy mit 1 bis 4 C-Atomen, Hydroxy,

b) Phenyl, gegebenenfalls mono- oder disubstituiert mit $C_1$-$C_4$-Alkyl, $C_1$-$C_4$-Alkoxy oder Halogen,

c) Nitro, Amino,

d) Carboxyl, Alkoxycarbonyl mit 1 bis 4 C-Atomen im Alkoxyteil oder Carbonamido der Formel 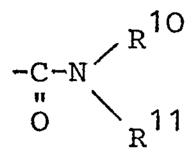

worin $R^{10}$ und $R^{11}$ gleich oder verschieden sind und Wasserstoff oder Alkyl mit 1 bis 6 C-Atomen bedeuten, wobei die Alkylreste gemeinsam mit dem Stickstoffatom einen 5- bis 7-gliedrigen Ring bilden können, worin eine $-CH_2$-Gruppe durch Sauerstoff, Stickstoff oder die $NR^{12}$-Gruppe, worin $R^{12}$ Wasserstoff, $C_1$-$C_4$-Alkyl, Phenyl oder Phenylalkyl mit 1 bis 2 C-Atomen im Alkylteil bedeutet, ersetzt sein kann,

Y Wasserstoff, $C_1$-$C_4$-Alkyl oder Phenyl

und von deren physiologisch verträglichen Salzen mit Säuren, dadurch gekennzeichnet, daß man

a) ein Epoxid der Formel II,

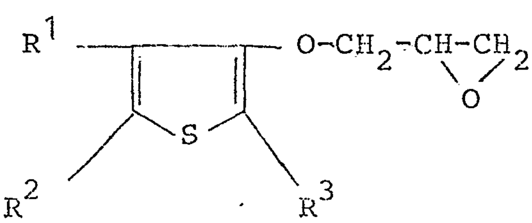

II

worin $R^1$, $R^2$ und $R^3$ die obige Bedeutung haben, mit einem Amin der Formel III,

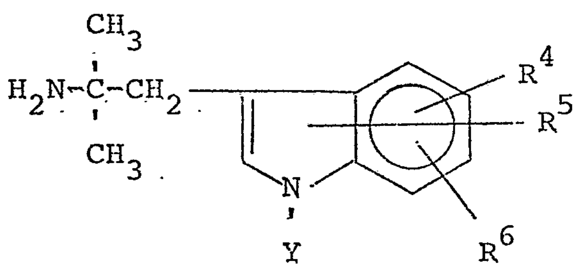

III

worin $R^4$, $R^5$, $R^6$ und Y die obige Bedeutung haben, umsetzt oder

b) eine Verbindung der Formel IV,

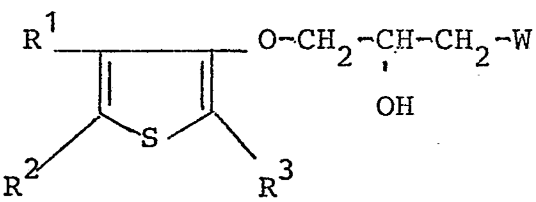

IV

worin $R^1$, $R^2$ und $R^3$ die obige Bedeutung haben und W eine durch nukleophile Substitution ersetzbare Gruppe darstellt, mit einem Amin der Formel III, worin $R^4$, $R^5$, $R^6$ und Y die obige Bedeutung haben, umsetzt, oder

c) ein Amin der Formel V,

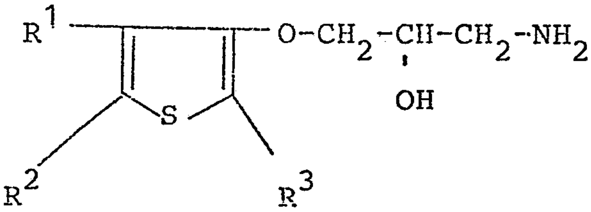

V

worin $R^1$, $R^2$ und $R^3$ die obige Bedeutung haben, mit einer Verbindung der Formel VI,

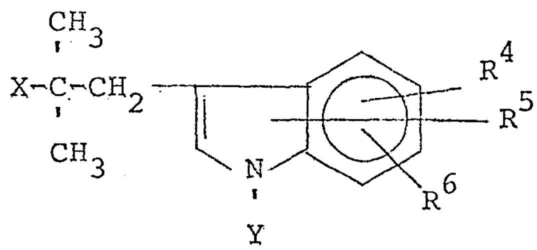

VI

worin $R^4$, $R^5$, $R^6$ und Y die obige Bedeutung haben und X eine durch nukleophile Substitution ersetzbare Gruppe darstellt, umsetzt, oder

d) ein Thiophenderivat der Formel VII,

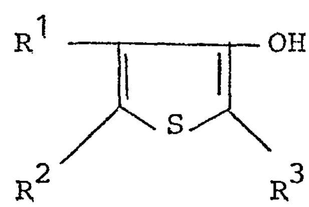

VII

worin $R^1$, $R^2$ und $R^3$ die obige Bedeutung haben, mit einer Verbindung der Formel VIII,

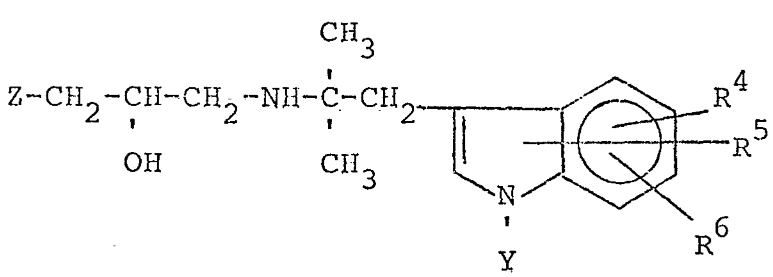

VIII

worin $R^4$, $R^5$, $R^6$ und Y die zu Formel I angegebene Bedeutung haben und Z eine durch nukleophiele Substitu-

tion ersetzbare Gruppe darstellt, in Gegenwart eines säurebindenden Mittels umsetzt, oder

e) in einer Verbindung der Formel IX

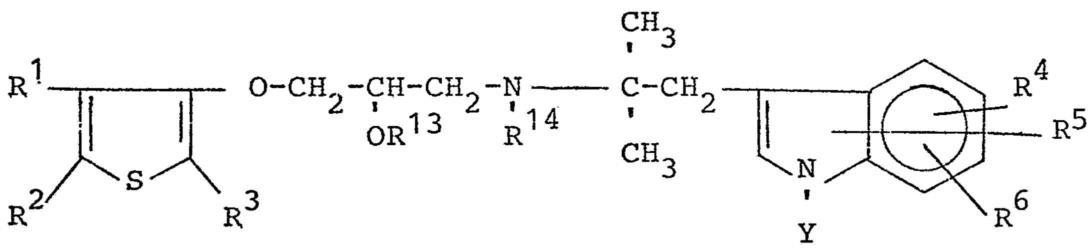

IX

worin $R^1$, $R^2$, $R^3$, $R^4$, $R^5$, $R^6$ und Y die obige Bedeutung haben, $R^{13}$ ein Wasserstoffatom, einen Acylrest oder den Benzylrest und $R^{14}$ ein Wasserstoffatom oder den Benzylrest bedeutet, $R^{13}$ und $R^{14}$ jedoch nicht beide gleichzeitig für Wasserstoff stehen können, die Benzylgruppe durch katalytische Hydrierung in Gegenwart von Edelmetallkatalysatoren abspaltet und/oder die Acylgruppe hydrolysiert

und

gegebenenfalls die erhaltenen basischen Verbindungen in üblicher Weise mit optisch aktiven Säuren zu Salzen umsetzt, aus den erhaltenen Salzen die optisch aktiven Basen in Freiheit setzt und die racemischen oder optisch aktiven Basen in an sich bekannter Weise in Salze mit physiologisch verträglichen Säuren überführt.

Europäisches Patentamt

# EUROPÄISCHER RECHERCHENBERICHT

0065295

Nummer der Anmeldung

EP 82 10 4244

| Kategorie | EINSCHLÄGIGE DOKUMENTE<br>Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | DE-A-2 720 613 (BASF)<br>* Insgesamt *<br>--- | 1-5 | C 07 D 409/12<br>A 61 K 31/40 |
| Y | FR-A-2 464 952 (ROUSSEL-UCLAF)<br>* Insgesamt *<br>--- | 1-5 | |
| Y | EP-A-0 025 727 (CM INDUSTRIES)<br>* Insgesamt *<br>--- | 1-5 | |
| Y | DE-A-2 830 884 (BRISTOL-MYERS)<br>* Insgesamt *<br>----- | 1-5 | |
| | | | RECHERCHIERTE SACHGEBIETE (Int. Cl. 3) |
| | | | C 09 D 409/00 |

Der vorliegende Recherchenbericht wurde für alle Patentansprüche erstellt.

| Recherchenort | Abschlußdatum der Recherche | Prüfer |
|---|---|---|
| DEN HAAG | 09-08-1982 | ALLARD M.S. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82